(19) Europäisches Patentamt
European Patent Office
Office européen des brevets

(11) Veröffentlichungsnummer : **0 394 798 B1**

# (12) EUROPÄISCHE PATENTSCHRIFT

(45) Veröffentlichungstag der Patentschrift :
**03.11.93 Patentblatt 93/44**

(51) Int. Cl.$^5$ : **A61K 6/093,** A61K 6/083

(21) Anmeldenummer : **90107182.9**

(22) Anmeldetag : **14.04.90**

## (54) Dentalmaterial (I).

(30) Priorität : **22.04.89 DE 3913252**

(43) Veröffentlichungstag der Anmeldung :
**31.10.90 Patentblatt 90/44**

(45) Bekanntmachung des Hinweises auf die Patenterteilung :
**03.11.93 Patentblatt 93/44**

(84) Benannte Vertragsstaaten :
**AT BE CH DE DK ES FR GB IT LI LU NL**

(56) Entgegenhaltungen :
**EP-A- 0 381 961**
**DE-A- 2 403 211**
**DE-A- 4 002 726**
**GB-A- 2 051 842**

(73) Patentinhaber : **Degussa Aktiengesellschaft**
**Weissfrauenstrasse 9**
**D-60311 Frankfurt (DE)**

(72) Erfinder : **Panster, Peter, Dr.**
**Im Lochseif 8**
**D-6458 Rodenbach (DE)**
Erfinder : **Janda, Ralf, Dr.**
**Seulberger Strasse 19**
**D-6380 Bad Homburg v.d.H. (DE)**
Erfinder : **Kleinschmit, Peter, Dr.**
**Wildaustrasse 19**
**D-6450 Hanau 9 (DE)**

**EP 0 394 798 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung bezieht sich auf ein pastöses, in Gegenwart eines Initiators zu einer auf Hochglanz polierbaren Masse aushärtbares Dentalmaterial aus einem polymerlslerbaren organischen Bindemittel und einem feinteiligen Füllstoff. Das Material enthält als Bindemittel mindestens ein polymerisierbares Methacrylat und einen gegebenenfalls silanisierbaren neuartigen Füllstoff auf Basis funktioneller Polysiloxane. Daneben können Initiatoren zur Polymerisationsauslösung, weitere Füllstoffe wie fein gemahlene Gläser, hochdisperse Kieselsäure oder vorgebildete Polymerisate, Pigmente und Stabilisatoren enthalten sein. Auch andere Additive wie Welchmacher oder Schlagzähigkeltsverbesserer kommen in Frage.

Der Begriff "Dentalmaterial" umfaßt beispielsweise Füllungsmaterialien, um kariöse Defekte oder andere Zahndefekte im Mundraum zu versorgen, Inlays, Kronen-und Brückenmaterialien, Verblendungen, Versiegelungs- und Schutzüberzugsmassen, Kunststoffbefestigungsmaterialien zum Festsetzen von Inlays oder Kronen und Brücken, Stumpfaufbaumaterialien, Prothesenmaterialien sowie Massen zur Herstellung von künstlichen Zähnen.

Übliche Dentalmassen der obengenannten Art enthalten mindesten seinen monomeren Ester der Methacrylsäure, meist aber ein Gemisch aus mehreren solcher Ester. Geeignete monofunktionelle Ester der Methacrylsäure sind beispielsweise Methylmethacrylat, Ethylmethacrylat, Isopropylmethacrylat, n-Hexylmethacrylat und 2-Hydroxyethylmethacrylat.

Neuerdings werden häufig auch mehrfunktionelle Ester der Methacrylsäure mit höherem Molekulargewicht eingesetzt, wie Ethylenglykoldimethacrylat, Butandiol-1,4-dimethacrylat, Triethylenglykoldimethacrylat, Dodecandiol-1,12-dimethacrylat, Decandiol-1,10-dimethacrylat, 2,2-Bis-[p(γ-methacryloxy-β-hydroxypropoxy)-phenyl ]-propan das Diadukt aus Hydroxyethylmethacrylat und Trimethylhexamethylendiisocyanat, das Diadukt aus Hydroxyethylmethacrylat und Isophorondiisocyanat, Trimethylolpropantrimethacrylat, Pentaerythrittrimethacrylat, Pentaerythrittetramethacrylat und 2,2-Bis[p(β-hydroxy-ethoxy)-phenyl]-propandimethacrylat (Bis-GMA).

Die Materialien für die dentale Anwendung können, je nach Anwendungszeck, auf unterschiedliche Weise ausgehärtet werden. Zahnfüllungsmaterialien gibt es sowohl als lichthärtende als auch als selbsthärtende (autopolymerisierende) Massen. Die lichthärtenden Massen enthalten Photoinitiatoren wie Benzoinalkylether, Benzilmonoketale, Acylphosphinoxide oder aliphatische und aromatlsche 1,2-Diketo-Verbindungen wie beispielsweise Campherchinon sowie Polymerisationsbeschleuniger wie aliphatische oder aromatische tertiäre Amine (z.B. N,N-Dimethyl-p-toluidin Triethanolamin) oder organische Phosphite und erhärten bei Bestrahlung mit UV- oder sichtbarem Licht.

Die selbsthärtenden Materialien bestehen in der Regel aus einer Katalysator- und einer Basispaste, von denen jede den Bestandteil eines Redoxsystems enthält und die beim Vermischen beider Komponenten polymerisieren. Der eine Bestandteil des Redoxsystems ist meistens ein Peroxid, wie beispielsweise Dibenzoylperoxid, der andere meistens ein tertiäres aromatlsches Amin, wie beispielsweise N,N'-Dimethyl-p-toluidin.

Andere Dentalmaterialien wie Prothesenkunststoffe oder Kunststoffmassen zur Herstellung künstlicher Zähne können unter Wärmeeinwirkung polymerisiert werden. Als Initiatoren dienen hier in der Regel Peroxide wie Dibenzoylperoxid, Dilaurylperoxid oder Bis(2,4-dichlor-benzoylperoxid).

Dentalmaterialien enthalten weiterhin in der Regel Pigmente, die - in geringer Menge zugesetzt - dazu dienen, die Farbe der Dentalmassen mit den verschiedenen Schattierungen natürlicher Zähne in Übereinstimmung zu bringen. Geeignete Pigmente sind beispielsweise Eisenoxidschwarz, Eisenoxidrot, Eisenoxidgelb, Eisenoxidbraun, Cadmiumgelb und -orange, Zinkoxid und Titandioxid.

Dentalmaterialien enthalten ferner meist organische oder anorganische Füllstoffe, Dies geschieht, um die Volumenschrumpfung der Kunststoffmasse bei der Polymerisation zu vermindern.

Reines monomeres Methylmethacrylat schrumpft beispielsweise bei der Polymerisation um ca. 20 Vol.%. Durch Zusatz von etwa 60 Gewichtsteilen festem Methylmethacrylat-Perlpolymerisat kann die Schrumpfung auf ca. 5 - 7 Vol.% reduziert werden (DE-PS 24 03 211).

Andere organische Füllstoffe werden erhalten, indem man ein Polymerisat herstellt, das im wesentlichen aus Estern der Methacrylsäure besteht und unvernetzt oder vernetzt ist. Gegebenenfalls enthält dieses Polymerisat oberflächenbehandelte Füllstoffe. Ist es als Polymerisat hergestellt, kann es dem Dentalmaterial in dieser Form zugesetzt werden; ist es dagegen durch Substanzpolymerlsation in kompakter Form hergestellt, so muß es vor der Einbringung in das Dentalmaterial erst zu einem sog. Splitterpolymerisat vermahlen werden.

Häufig verwendete vorgebildete Polymerisate sind neben den bereits erwähnten füllstoffhaltigen Perl- und Splitterpolymerisaten Homopolymerisate des Methacrylsäuremethylesters oder, vorzugsweise unvernetzte, Copolymerisate des Methacrylsäuremethylesters mit einem geringen Anteil an Estern der Methacrylsäure oder Acrylsäure mit 2 bis 12 C-Atomen in der Alkoholkomponente, zweckmäßigerweise in der Form eines Perlpolymerisates. Andere geeignete Polymerisate sind unvernetzte Produkte auf der Basis von Polyurethanen, Polycarbonaten, Polyestern und Polyethern.

Anorganische Füllstoffe sind beispielsweise gemahlene Gläser oder Quarz mit mittleren Teilchengrößen zwischen etwa 1 und 10 μm sowie hochdisperses $SiO_2$ mit mittleren Teilchengrößen zwischen etwa 10 - 400 nm.

Bei den Gläsern handelt es sich vorzugsweise um Aluminiumsilikatglaser, die mit Barium, Strontium oder seltenen Erden dotiert sein können (DE-PS 24 58 380).

Hinsichtlich des feingemahlenen Quarzes bzw. der feingemahlenen Gläser sowie des hochdispersen $SiO_2$ bleibt anzumerken, daß der anorganische Füllstoff in der Regel vor dem Vermischen mit den Monomeren zwecks besserer Bindung an die organische Matrix silanisiert wird. Hierfür werden die anorganischen Füllstoffe mit Silankupplungsmitteln überzogen, die meistens eine polymerisierbare Doppelbindung zur Reaktion mit den monomeren Estern der Methacrylsäure aufweisen.

Geeignete Silankupplungsmittel sind beispielsweise Vinyltrichlorsilan, Tris-(2-methoxyethoxy)-vinylsilan, Tris-(acetoxy)-vinylsilan und 3-Methacryloyloxy-propyltrimethoxysilan.

Auch die eingangs erwähnten, neuerdings verwendeten Monomere mit höherem Molekulargewicht bewirken ebenfalls eine Verringerung der Polymerisationsschrumpfung. Diesen Monomeren werden nun bis zu etwa 85 Gew.% die beschriebenen inerten anorganischen feingemahlenen Gläser oder organischen Füllstoffe oder Gemische aus diesen zugesetzt, wodurch eine weitere Reduzierung der Schrumpfung auf ca. 1 Vol.% erreicht werden kann.

Die anorganischen Füllstoffe bewirken nicht nur eine Verminderung der Polymerisationsschrumpfung, sondern darüber hinaus auch eine erhebliche Verstärkung des organischen Polymergefüges.

Diese Verstärkung macht sich in einer Verbesserung der mechanischen Eigenschaften sowie in einer Erhöhung der Abriebsfestigkeit deutlich bemerkbar (R. Janda, Quintessenz 39, 1067, 1243, 1393 (1988). Gute mechanische Eigenschaften und hohe Abriebsfestigkeit sind wichtige Forderungen, denen eine Dentalmasse, die verlorene Zahnhartsubstanz dauerhaft ersetzen soll, gerecht werden muß.

Neben den verstärkenden Eigenschaften müssen die Füllstoffe aber ebenfalls auch anderen Materialparametern gerecht werden. Ein wesentlicher Parameter in diesem Zusammenhang ist die Polierbarkeit.Hochglanzpolierbarkeit ist für Füllungsmaterialien und Kronen- und Brückenmaterialien aus mindestens zwei Gründen von erheblicher Bedeutung:

- Aus ästhetischen Gründen ist vom Füllungsmaterial eine hochglänzende und völlig homogene Oberfläche zu fordern, damit die Füllung vom umgebenden, absolut glatten, natürlichen Zahnschmelz nicht mehr zu unterscheiden ist. Weiterhin muß diese hochglänzende Füllungsoberfläche ihren Charakter auch langfristig beibehalten.
- Eine hochglatte Füllungsoberfläche ist auch deshalb wichtig, damit Plaque oder verfärbende Medien keine mechanischen Verankerungsstellen vorfinden.

Nun hat es sich aber gezeigt, daß die vorstehend beschriebenen feingemahlenen Quarz- oder Glasfüllstoffe zwar gute verstärkende Eigenschaften besitzen, jedoch hinsichtlich ihrer Polierbarkeit nicht den Anforderungen entsprechen. Daher hat man versucht, diese anorganischen Füllstoffe noch feiner zu mahlen, um homogenere Oberflächen zu erhalten. Den physikalischen Mahlmethoden sind allerdings Grenzen gesetzt, so daß mittlere Korngrößen unter 1 μm nur noch sehr schwer zu erzeugen sind.

Als man hochdisperse Kieselsäure (mittlere Teilchengröße 10 - 400 nm) als Füllstoff in Dentalmassen verwendete (DE-PS 24 03 211), zeigte sich überraschenderweise, daß mit diesen Füllstoffen eine erhebliche Verbesserung der Polierbarkeit erreicht werden konnte. Nachteile der hochdispersen Kieselsäure bestehen aufgrund ihrer starken verdickenden Wirkung, so daß heute in der Regel Füllgrade über 52 Gew.% nicht erreicht werden können, es sei denn, man begnügt sich mit unzureichenden verarbeitungstechnischen Eigenschaften.

Darüber hinaus zeigen die mit hochdisperser Kieselsäure gefüllten Materialien deutlich geringere Festigkeiten und Härten als die mit Quarz oder feingemahlenen Gläsern gefüllten.

Aufgabe der Erfindung ist, neue licht-, heiß- oder selbsthärtende Dentalmaterialien aus einem polymerisierbaren organischen Bindemittel und einem feinteillgen Füllstoff bereitzustellen, die einerseits auf Hochglanz polierbar sind und damit den ästhetischen Anforderungen eines Dentalwerkstoffes genügen, andererseits aber bessere physikalische Eigenschaften besitzen als die den momentanen Stand der Technik repräsentierenden polierbaren Dentalmaterialien.

Diese Aufgabe wurde dadurch gelöst, daß von einem pastösen, in Gegenwart eines Initiators zu einer auf Hochglanz polierbaren Masse aushärtbaren Dentalmaterial aus einem polymerisierbaren organischen Bindemittel, enthaltend mindestens ein polymerisierbares Methacrylat und 20 bis 90 Gew.-%, bezogen auf das Gesamtgewicht, eines feinteiligen Füllstoffs ausgegangen wird, welcher aus einem Organosiloxan-Copolykondensat als statistisches Copolykondensat, Block-Copolykondensat oder als Gemisch aus diesen Formen bestehend vorliegt und eine spezifische Oberfläche von bis zu 200 $m^2/g$ und eine Partikelgröße von 0,01 μm bis

100 µm aufweist und aus Einheiten der Formeln

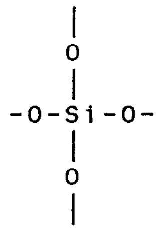 (I)

und

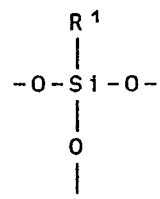 (II)

wobei $R^1$ für eine mit einem Acrylat- oder Methacrylatrest verbundene lineare, gegebenenfalls verzweigte Alkylgruppe mit 1 bis 6 C-Atomen oder für einen einfach olefinisch ungesättigten, vorzugsweise endständig ungesättigten linearen, gegebenenfalls verzweigten Kohlenwasserstoffrest mit 2 bis 8 C-Atomen oder für einen zyklischen, einfach olefinisch ungesättigten Kohlenwasserstoffrest mit 5 - 8 C-Atomen oder für eine lineare, gegebenenfalls verzweigte Alkylgruppe mit 1 bis 8 C-Atomen, eine Phenylgruppe, eine Cycloalkylengruppe mit 5 bis 8 C-Atomen oder eine Alkylarylgruppe steht und/oder Einheiten der Formel

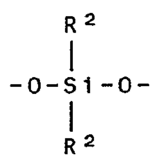 (III)

in denen $R^2$ eine Methyl-, Ethyl- oder Phenylgruppe repräsentiert, aufgebaut ist und die freien Valenzen der an die Siliciumatome gebundenen Sauerstoffatome bei den Einheiten (I), (II) und (III) wie bei den Kieselsäuregerüsten durch ein Siliciumatom einer gleichen oder einer unterschiedlichen Einheit abgesattigt werden, wobei das Verhältnis der Siliciumatome aus den Einheiten der Formel (I) zu der Summe der Siliciumatome der Einheiten (II) und (III) 3 : 1 bis 100 : 1 beträgt und das erhältlich ist

A) in Gestalt eines statistischen Copolykondensats, indem man

a) ein Alkoxysilan der allgemeinen Formel

$$Si(OR^3)_4 \qquad (IV)$$

wobei $R^3$ für eine lineare oder verzweigte Alkylgruppe mit 1 bis 5 C-Atomen steht und ein Alkoxysilan der allgemeinen Formel

$$R^1 - Si(OR^3)_3 \qquad (V)$$

in der $R^1$ wie unter Formel (II) und $R^3$ wie unter Formel (IV) definiert sind und/oder ein Alkoxysilan der allgemeinen Formel

$$R^2_2Si(OR^3)_2 \qquad (VI)$$

in der $R^2$ wie unter Formel (III) und $R^3$ wie in Formel (IV) definiert sind, in einem weitgehend wassermischbaren, aber die Silane nach Formeln (IV) bis (VI) lösenden Lösungsmittel auflöst und der Lösung unter Rühren eine zumindest für die vollständige Hydrolyse und Kondensation ausreichende Menge Wasser zufügt und das Reaktionsgemisch unter Weiterrühren bei einer bestimmten Temperatur im Bereich von Raumtemperatur bis 200° C vorkondensiert,

b) den sich bildenden Feststoff gegebenenfalls nach Zusatz weiteren Lösungsmittels oder Wassers noch 1 Stunde bis zu 6 Stunden bei 60° C bis 200° C, bei Normaldruck oder einem Druck rührt, welcher

der Summe der Partialdrucke bei der jeweiligen Temperatur entspricht,

c) das als Feststoff gebildete Organopolysiloxan nach gängigen Techniken von der flüssigen Phase abtrennt, gegebenenfalls wäscht,

d) dann in Wasser, einem Wasser/Alkohol-Gemisch oder in reinem Alkohol, in Gegenwart eines sauren oder eines basischen Katalysators nachkondensiert und

e) gegebenenfalls bei Raumtemperatur bis 200° C, gegebenenfalls unter Schutzgasatmosphäre oder im Vakuum trocknet, gegebenenfalls 1 bis 100 Stunden bei Temperaturen von 150° C bis 250° C unter Schutzgasatmosphäre oder im Vakuum tempert und gegebenenfalls mahlt und/oder klassifiziert.

B) in Gestalt eines Block-Copolykondensats, indem man

a) die monomeren Komponenten nach Formel (IV), (V), und/oder (VI) jeweils unabhängig voneinander, ohne oder unter Einsatz eines die Ausgangsstoffe lösenden Lösungsmittels in Gegenwart einer nicht zur vollständigen Hydrolyse ausreichenden Menge Wasser über einen Zeitraum von 5 Minuten bis zu 5 Tagen bei Raumtemperatur bis 200° C vorkondensiert, die erhaltenen Vorkondensate vereinigt und gegebenenfalls nach Zusatz weiteren Wassers und/oder weiteren Lösungsmittels die unter A) genannten Schritte b) bis e) anschließt oder

C) in Gestalt eines gemischten Copolykondensats, indem man

a) von den monomeren Komponenten nach Formel (IV), (V) und/oder (VI) mindestens ein Monomer aber höchstens 2 Monomere, voneinander unabhängig wie bei B) vorkondensiert, das erhaltene Vorkondensat oder die erhaltenen Vorkondensate und mindestens eine nicht vorkondensierte Komponente miteinander vereinigt und die unter A) genannten Schritte b) bis e) anschließt und

das dadurch gekennzeichnet its, daß man bei der Herstellung der Füllstoffe die Nachkondensation im Autoklaven über einen Zeitraum von 1 Stunde bis zu 5 Tagen bei Temperaturen von 60 bis 250° unter einem Druck, welcher der Summe der Partialdrucke bei der jeweiligen Temperatur entspricht, durchführt.

In der älteren, nachveröffentlichten deutschen Patentanmeldung gemaß DE-OS 39 03 407 ist zwar schon ein dentales Füllmaterial vorgeschlagen worden, für welches die im Oberbegriff des hier geltenten Patentanspruchs 1 aufgeführte Zusammensetzung im wesentlichen angegeben ist. Dort ist jedoch noch nicht die hier als erfindungsgemäß beanspruchte Überdruckbehandlung in flüssiger Phase zur Nachkondensation mit spezifischen Angaben zu Zeit und Temperatur vorgesehen, die zu einer Verfestigung der Struktur der eingesetzten Polymeren und zu einer beträchtlichen Verbesserung der mechanischen Eigenschaften führt.

Die Einheiten nach Formeln (I) bis (III) können natürlich in unterschiedlicher Form zueinander vorliegen, d. h. sie können in Form eines statistischen Copolykondensates oder in Form eines Block-Copolykondensates oder in Form eines sog. gemischten Copolykondensates vorliegen.

Erfindungsgemäß können die Füllstoffe der neuen Dentalmaterialien in bezug auf die Einheiten nach Formel (I) bis (III) in jeder der genannten Formen und ihrer Gemische vorliegen.

Dies bedeutet, daß im Fall eines reinen statistischen Copolykondensates, das Einheiten der Formel (I), (II), und/oder (III) enthält, eine rein statistische Verteilung der Komponenten entsprechend der molaren Verhältnisse der Ausgangsprodukte gegeben ist.

Im Fall eines sog. Block-Copolykondensates liegt eine Bildung von Blöcken gleicher Einheiten nach Formel (I) und (II) und/oder (III) vor. Schließlich weist ein sog. gemischtes Copolykondensat sowohl Strukturen eines statistischen Copolykondensates als auch eines Block-Copolykondensates auf.

Die erfindungsgemäßen Füllstoffe werden in den Dentalmassen in einer Menge von 20 bis 90 Gew.%, vorzugsweise 50 bis 85 Gew,%, eingesetzt. Der an den Einheiten nach Formel (II) gegebenenfalls vorhandene ungesättigte organische Rest $R^1$ kann vor allem dazu dienen, eine festere Anbindung des Polysiloxan-Füllstoffs an die später aus dem polymerisierbaren organischen Bindemittel erzeugte Polymermatrlx zu erreichen.

Besonders geeignet sind deshalb organische Reste $R^1$, bei denen eine Doppelbindung sterisch leicht zugänglich und vor allem auch relativ leicht zu polymerisieren ist. Dies trifft insbesondere zu für die Gruppe

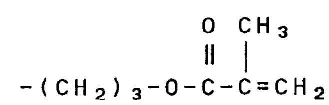

weil deren besonders leichte Polymerisierbarkeit bekannt ist und es sich daneben bei der Polymermatrix, in die der Füllstoff einzubringen ist, in der Regel um ein Methacrylatsystem handelt sowie für lineare Kohlenwasserstoffreste mit endständiger Doppelbindung, wie z. B. dem Vinyl-Butenyl- oder Octenylrest. Aber auch zyklische Kohlenwasserstoffreste mit polymerisierbaren Doppelbindungen sind geeignet. In vielen Fallen kann $R^1$ aber eine der ebenfalls unter Formel (II) in Anspruch 1 genannten doppelbindungsfreien organischen Reste

sein.

Eine besonders vorteilhafte Füllstoff-Zusammensetzung, die sich durch einfache Realisierbarkeit und vor allem durch die technische Verfügbarkeit der Ausgangsmaterialien auszeichnet, sieht vor, daß ein Organopolysiloxan als Füllstoff verwendet wird, das nur aus den Einheiten der Formel (I) und den speziellen Einheiten der Formel (II)

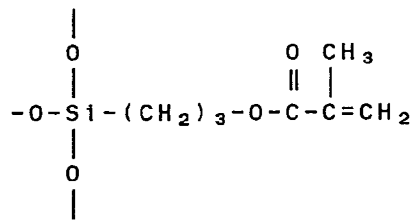

besteht, wobei das molare Verhältnis der Einheiten der Formel (I) zu den Einheiten der Formel (II) 3 : 1 bis 100 : 1 beträgt. Ein derartiger Füllstoff zeichnet sich dadurch aus, daß er zur Einbringung in die Methacrylatmatrix prinzipiell nicht mehr silanisiert, d.h. mit einem Methacrylsilan behandelt werden muß, nachdem diese Methacrylgruppen bereits in dem Füllstoff homogen verteilt vorhanden sind.

Dies schließt jedoch nicht aus, daß im Einzelfall, im Hinblick auf eine weitere Hydrophobierung mit weiterer Verstärkung der Anbindung zwischen dem OrganopolysiloxanFüllstoff und organischer Polymermatrix, zusätzlich eine Silanisierung des Füllstoffs vorgenommen wird.

Überraschenderwelse hat sich bei der Ausarbeitung der Erfindung herausgestellt, daß sehr gute mechanische Eigenschaften und Polierbarkeit des Dentalmaterials auch dann erreicht werden können, wenn der verwendete Organosiloxan-Füllstoff keine ungesättigten, sondern nur gesättigte Reste $R^1$ enthält.

Dies gilt sowohl für Füllstoffe, die Einheiten nach Formel (I), (II) und (III) als auch für solche Füllstoffe, die nur Einheiten nach Formel (I) und (II) enthalten. Derartige nicht-doppelbindungsfunktionelle Organopolysiloxan-Füllstoffe sollten vor ihrer Einbringung in die organische Polymermatrix mit einer geeigneten Organosilanverbindung, vorzugsweise 3-Methacryloyloxy-propyltrimethoxy- oder 3-Methacryloyloxypropyltriethoxysilan, behandelt werden.

Analoges gilt auch für eine, aus Gründen der besonders leichten Verfügbarkeit der Ausgangsmaterialien vorteilhafte erfindungsgemäße Füllstoffzusammensetzung, die einen Aufbau des Polysiloxans aus Einheiten der Formel (I) und den speziellen Einheiten der Formel (III)

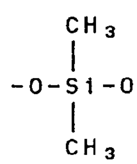

vorsieht, wobei das molare Verhältnis der Einheiten nach Formel (I) zu den Einheiten der Formel (III) 3 : 1 bis 100 : 1 beträgt.

Die monomeren Bausteine der erfindungsgemäßen Füllstoffe sind prinzipiell bekannte Verbindungen, beispielsweise Si $(OC_2H_5)_4$ als Monomerbaustein für eine Einheit der Formel (I), eine Verbindung

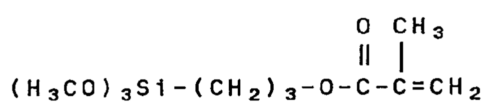

bzw.

$$(H_3CO)_3Si\text{-}CH_2CH_2CH_3$$

als Monomerbausteine für Einheiten der Formel (II) und eine Verbindung $(H_3C)_2Si(OC_2H_5)_2$ als Monomerbaustein für Einheiten der Formel (III).

Die Zusammensetzungen der daraus herstellbaren erfindungsgemäßen Dentalfüllstoffe lassen sich z.B. durch Formeln für eine jeweilige Polymereinheit wie

```
                  CH3
                  |
40 SiO2·CH2=C-C-O-(CH2)3SiO3/2·(CH3)2SiO2/2
                    ||
                    O
```

$10\ SiO_2 \cdot C_3H_7SiO_{3/2} \cdot (H_5C_2)_2SiO_{2/2}$

$50\ SiO_2 \cdot (H_3C)_2SiO_{2/2}$

oder

```
                  CH3
                  |
30 SiO2·CH2=C-C-O-(CH2)3SiO3/2
                    ||
                    O
```

beschreiben.

Ein typischer saurer Katalysator ist zum Beispiel Salzsäure oder Essigsäure, während zum Beispiel neben Ammoniak Amine einen typischen Basenkatalysator repräsentieren.

Im Hinblick auf die physikalischen Eigenschaften besitzen Füllstoffe der erfindungsgemäßen Zusammensetzung dann eine besonders gute Eignung zur Verwendung in den erfindungsgemäßen Dentalmaterialien, wenn sie eine spezifische Oberfläche bis 200 $m^2/g$, vorzugsweise bis 100 $m^2/g$, und eine Partikelgröße von 0,01 - 100 µm vorzugsweise 0,1 µm bis 30 µm, aufweisen.

Die in dem erfindungsgemäßen Dentalmaterial enthaltenen Füllstoffe sind nach den Methoden A), B) und C) erhältlich.

Bevorzugt ist der Füllstoff des Dentalmaterials in Gestalt eines statistischen Copolymerisats erhältlich, indem Hydrolyse und Kondensation in Methanol, Ethanol n- und i-Propanol, n- und i-Butanol und/oder n-Pentanol durchgeführt werden.

Bevorzugt löst man zur Herstellung eines Block-Copolykondensats oder eines gemischten Copolykondensats die Ausgangsstoffe in einem zu deren Alkoxygruppen korrespondierenden, linearen oder verzweigten Alkohol mit 1 bis 5 C-Atomen.

Bevorzugt setzt man bei der Herstellung eines Block-Copolykondensats oder eines gemischten Copolykondensats im Schritt a) Wasser in einer Menge von 1 bis 100 Mol% der zur vollständigen Hydrolyse benötigten Menge ein.

Die vorteilhaften anwendungstechnischen Eigenschaften der neuen Füllstoffe gehen auch auf die saure oder alkalische Temperaturbehandlung vor oder nach Trocknung oder in einer der gegebenenfalls noch angewandten Behandlungsstufen zurück, da dadurch vor allem eine Festigung der Polymerstruktur erreicht wird.

Prinzipiell können als Ausgangsstoffe für das Verfahren anstelle der Alkoxyverbindungen auch die entsprechenden Halogenid- oder Phenoxyverbindungen eingesetzt werden, doch bietet deren Verwendung keine Vorteile, sondern kann zum Beispiel im Fall der Chloride, Schwierigkeiten durch die bei der Hydrolyse freiwerdende Salzsäure verursachen.

Die Hydrolyse der Monomeren muß in einem weitgehend wassermischbaren, aber die Ausgangsstoffe lösenden Lösungsmittel durchgeführt werden. Bevorzugt werden Alkohole verwendet, die zu den Alkoxygruppierungen an den monomeren Ausgangsstoffen korrespondieren.

Besonders geeignet sind Methanol, Ethanol, n- und i-Propanol, n- und i-Butanol und n-Pentanol. Auch Gemische solcher Alkohole können als Lösungsmittel bei der Hydrolyse eingesetzt werden.

Anstelle von Alkoholen können natürlich auch andere polare Lösungsmittel, die weitgehend wassermischbar sind, verwendet werden, doch erweist sich dies aus verfahrenstechnischen Gründen wegen der mit dem hydrolytisch abgespaltenen Alkohol zustandekommenden Lösungsmittelgemische als nicht sinnvoll.

Bevorzugt führt man die Hydrolyse mit einem Überschuß an Wasser über die stöchiometrisch erforderliche Menge durch. Die zur Hydrolyse benötigte Menge Wasser hängt von der Hydrolysegeschwindigkeit der jeweils verwendeten Monomere in der Weise ab, daß mit zunehmender Menge Wasser raschere Hydrolyse erfolgt, allerdings kann eine Obergrenze durch auftretende Entmischung und Ausbildung eines Zweiphasensystems vorgegeben sein. Grundsätzlich ist eine Hydrolyse in homogener Lösung vorzuziehen.

Aufgrund der genannten Aspekte wird in der Praxis gewichtsmäßig maximal die Menge Wasser verwendet, wie an Silanmonomeren insgesamt eingesetzt wird.

Die Polykondensation kann bei verschiedenen Temperaturen durchgeführt werden. Nachdem die Polykondensation bei höheren Temperaturen am schnellsten verläuft, wird diese bevorzugt bei Rückflußtemperatur oder knapp darunter durchgeführt. Prinzipiell kann die Hydrolyse und Polykondensation bei noch höherer Temperatur als Rückflußtemperatur, d. h. unter Druck, durchgeführt werden.

Bei der Polykondensation kann das Reaktionsgemisch zu einer festen Masse erstarren. Aus diesem Grunde ist es angebracht, eine entsprechende Menge Lösungsmittel oder Wasser zur Verdünnung zuzusetzen.

Das Lösungsmittel wird dabei in der Regel dasselbe sein, das schon bei der Hydrolyse der Silane eingesetzt wurde, d. h. bevorzugt wird ein niederer Alkohol mit 1 bis 5 C-Atomen verwendet.

Alternativ zu der Verdünnung mit einem Lösungsmittel kann natürlich auch mit Wasser verdünnt werden. Was im Einzelfall verwendet wird, hängt auch davon ab, welche physikalischen Eigenschaften das herzustellende Copolykondensat haben soll.

Auch durch die Dauer und Temperatur der Nachbehandlung in flüssiger Phase oder in trockener Form kann hierauf Einfluß genommen werden. Eine Nachreaktion bei höherer Temperatur führt durchwegs zu einer Verfestigung der Struktur des Polymeren und zu einer Verbesserung der mechanischen Eigenschaften.

Die Abtrennung des gebildeten Feststoffs kann nach gängigen Techniken wie Filtrieren, Dekantieren, Zentrifugieren oder durch Abdestillieren der flüssigen Phase erfolgen. Die Waschung des gebildeten Feststoffs wird bevorzugt mit dem bei der Fällung verwendeten Lösungsmittel oder mit Wasser durchgeführt.

Das getrocknete bzw. getemperte Produkt kann in üblichen Vorrichtungen gemahlen und in verschiedene Korngrößenfraktionen klassifiziert werden. Von den Aufarbeitungsmaßnahmen Waschung, Trocknung, Temperung, Mahlung und Klassifizierung kann, je nach Umständen, die eine oder andere entfallen oder in einer anderen Reihenfolge durchgeführt werden.

Eine Klassifizierung kann zum Beispiel auch an flüssigkeitsfeuchtem, gegebenenfalls vorher getrocknetem oder getempertem Produkt durchgeführt werden.

Die Dauer der Hydrolyse hängt von der Hydrolyseeignung der Ausgangsstoffe und von der Temperatur ab. Die Hydrolysegeschwindigkeit hängt insbesondere von den Silicium-ständigen Alkoxygruppen ab, wobei die Methoxygruppe am schnellsten hydrolysiert und mit steigender Kettenlänge oder zunehmender Verzweigung eine Verlangsamung eintritt.

Hydrolyse und Polykondensation können durch den Zusatz von organischen oder anorganischen Basen, wie z. B. Ammoniak oder Aminen, oder organischen oder anorganischen Säuren, wie z. B. Salzsäure, oder aber von üblichen Kondensationskatalysatoren, wie z. B. Dibutylzinndiacetat, beschleunigt werden.

Um ein unterschiedliches Hydrolyse- und Polykondensationsverhalten der monomeren Komponenten eines statistischen Copolykondensates auszugleichen, können nach einer Herstellvariante die monomeren Komponenten nach Formel (IV), (V) und/oder (VI) vorkondensiert werden. Hierzu werden diese monomeren Komponenten ohne oder unter Verwendung eines die Ausgangsstoffe lösenden Lösungsmittels, bevorzugt sind zu den Alkoxygruppen korrespondierende lineare oder verzweigte Alkohole mit 1 bis 5 C-Atomen, in Gegenwart einer nicht zur vollständigen Hydrolyse ausreichenden Menge Wasser, vorzugsweise 1 bis 100 Mol% der hierzu benötigten Menge, über einen Zeitraum von 5 Minuten bis zu 5 Tagen bei Raumtemperatur bis 200° C vorkondensiert.

Um diesen Vorkondensationseffekt zu begünstigen, kann dabei ein saurer oder basischer Kondensationskatalysator zugesetzt werden. Bevorzugte Katalysatoren sind zum Beispiel Salzsäure, Essigsäure, Ammoniak, Natronlauge oder Kalilauge, die gasförmig oder gelöst in Wasser oder einem organischen Lösungsmittel eingesetzt werden.

Nach erfolgter Vorkondensatlon wird die vollständige Hydrolyse und Polykondensation, gegebenenfalls nach Zusatz weiteren Wassers und gegebenenfalls nach Zusatz weiteren Lösungsmittels, wie beschrieben, durchgeführt.

Bevorzugt setzt man bei der Herstellung des Copolykondensats als basischen Katalysator für die Nachkondensation im Schritt d) Ammoniak ein.

Bei der Durchführung einer Vorkondensation hängt die verwendete Menge Wasser davon ab, welcher Oligomerisierungsgrad, d. h. welche Blockgröße erreicht werden soll. Bei Einsatz von mehr Wasser zur Vorkondensation und/oder längerer Vorkondensationszeit entstehen natürlich grundsätzlich größere Einheiten als bei Verwendung von weniger Wasser und/oder kürzerer Reaktionszeit. Die Dauer der Vorkondensation hängt, wie bereits vorstehend beschrieben, generell natürlich auch von der Hydrolysebereitschaft der monomeren Komponenten und der Temperatur ab.

Charakterisiert sind die Füllstoffe für die neuen Dentalmaterialien insbesondere anhand der quantitativen Hydrolyse- und Kondensationsausbeute und der Elementaranalysen. Zwischen den nach den unterschiedlichen Herstellungsverfahren erhaltenen Copolykondensaten besteht rein optisch kein Unterschied.

Je nach Behandlung besitzen die erfindungsgemäßen Füllstoffe Oberflächen bis 200 $m^2/g$. Die gewünschten Teilchengrößendurchmesser von 0,01 µm bis 100 µm lassen sich problemlos durch Anwendung gängiger Mahltechniken einstellen.

Ein weiterer Gegenstand der Erfindung ist die Verwendung des Dentalmaterials nach vorstehenden Ansprüchen zur Fertigung von Zahnfüllungen, Inlays, Zahnversiegelungen, Überzügen zum Schutz von Zahnoberflächen, Kronen, Verblendungen, Brücken, Zahnprothesen, künstlichen Zähnen, Klebern zur Befestigung von Inlays, Kronen und Brücken sowie zum Aufbau von Zahnstümpfen.

Die Erfindung wird nachfolgend anhand von Ausführungsbeispielen weiter erläutert.

**I. Herstellung der erfindungsgemäß einzusetzenden Füllstoffe:**

Beispiel 1

1.465,2 g (7.03 Mol) $Si(OC_2H_5)_4$ und 34.8 g (0,234 Mol) $(CH_3)_2\,Si(OC_2H_5)_2$ wurden in 750 ml Ethanol gelöst. Die Lösung wurde auf Rückflußtemperatur aufgeheizt und dann mit 525 ml 10 %iger wäßriger Ammoniaklösung versetzt. Es wurde zunächst eine halbe Stunde unter Rückfluß gerührt und dann der gebildete Feststoff mit 750 ml Wasser verdünnt. Nach weiteren 2 Stunden Rühren unter Rückfluß wurde die Suspension auf Raumtemperatur abgekühlt und der gebildete weiße Feststoff von der flüssigen Phase abfiltriert und mit insgesamt 500 ml Wasser gewaschen. Der Feststoff wurde mit 700 ml 2 %iger $NH_3$-Lösung versetzt und dann in einen Autoklaven überführt. Nach 24-stündiger Temperaturbehandlung des Autoklaveninhalts bei 150° C wurde der Feststoff 24 Stunden bei 120° C im Trockenschrank unter $N_2$-Atmosphäre getrocknet und dann 24 Stunden in einer Kugelmühle gemahlen.

Es wurden 437.2 g (99,4 % der Theorie) eines Dentalfüllstoffes. bestehend aus Polymereinheiten der Formel $(CH_3)_2SiO_{2/2} \cdot 30SiO_2$ erhalten.

Spezifische Oberfläche: 22 $m^2/g$

| Analysen: | % C | % H | % Si |
|---|---|---|---|
| Theorie: | 1,28 | 0,32 | 46,4 |
| Gefunden: | 1,12 | 0,40 | 45,6 |

Beispiel 2

1.456,5 g (6.99 Mol) $Si(OC_2H_5)_4$ und 43.5 (0.175 Mol) Methacryloyloxypropyltrimethoxysilan wurden in 750 ml Ethanol vereinigt. Die Lösung wurde in einem 6 l Dreihalskolben mit KPG-Rührer und Rückflußkühler unter Rühren auf Rückflußtemperatur aufgeheizt und in der Siedehitze mit 500 ml 5 %iger $NH_3$-Lösung versetzt. Nach ca. einer halben Stunde Rühren unter Rückfluß wurde der gebildete weiße Feststoff mit 750 ml Wasser verdünnt. Es wurde noch 5 Stunden unter Rückfluß gerührt. dann die Suspension auf Raumtemperatur abgekühlt und der Feststoff von der flüssigen Phase abfiltriert. Der Feststoff wurde mit 500 ml 5 %iger Ammoniaklösung versetzt und dann in einen Autoklaven überführt.

Nach 48-stündigem Rühren des Autoklaveninhalts bei 130° C wurde der Feststoff Ammoniak-frei gewaschen. dann 24 Stunden bei 100° C unter $N_2$-Atmosphäre getrocknet und schließlich 12 Stunden in einer Kugelmühle gemahlen. Erhalten wurden 450 g (99,6 % der Theorie) eines Dentalfüllstoffs. bestehend aus Einheiten der Formel

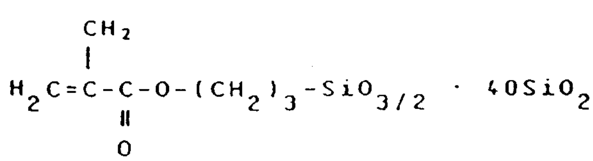

erhalten.

Spezifische Oberfläche: 23 $m^2/g$

| Analysen: | % C | % H | % Si |
|---|---|---|---|
| Theorie: | 3,26 | 0,43 | 44,6 |
| Gefunden: | 3,12 | 0,34 | 44,0 |

Beispiel 3

1.431.8 g (6.87 Mol) $Si(OC_2H_5)_4$, 42,7 g (0,172 Mol) Methacryloxypropyltrimethoxysilan und 25,5 g $(CH_3)_2Si(OC_2H_5)_2$ wurden in 750 ml Methanol vereinigt. Die Lösung wurde auf Rückflußtemperatur aufgeheizt und dann mit 400 ml 5 %iger Ammoniaklösung versetzt. Nach weiterer Verfahrensweise analog zu Beispiel 2 wurden 450 g (98.6 % der Theorie) eines Dentalfüllstoffs, bestehend aus Einheiten der Formel

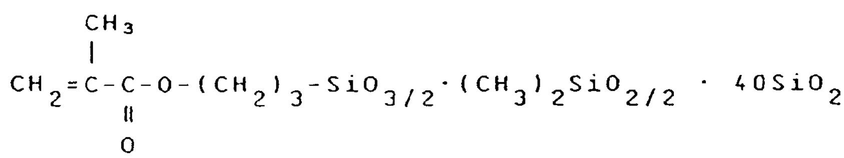

erhalten.

Spezifische Oberfläche: 32 $m^2/g$

| Analysen: | % C | % H | % Si |
|---|---|---|---|
| Theorie: | 4,07 | 0,64 | 44,4 |
| Gefunden: | 3,82 | 0,59 | 43,4 |

Beispiel 4

722,9 g (3,47 Mol) $Si(OC_2H_5)_4$, 14,3 g (0.0867 Mol) $n\text{-}C_3H_7\text{-}Si(OCH_3)_3$ und 12,9 g (0,0867 Mol) $(CH_3)_2Si(OC_2H_5)_2$ wurden in 375 ml Ethanol gelöst. Die Lösung wurde auf Rückflußtemperatur aufgeheizt und mit 200 ml ln-HCl-Lösung versetzt. Der nach 1 Stunde gebildete Feststoff wurde mit 400 ml Methanol verdünnt und noch eine halbe Stunde weiter unter Rückfluß gerührt. Der anschließend abfiltrierte Feststoff wurde mit 500 ml 10 %iger Ammoniaklösung versetzt und 15 Stunden in einem Autoklaven bei 150°C gerührt. Nach weiterer Aufarbeitung analog zu Beispiel 2 wurden 220,0 g (98,6 % der Theorie) eines Dentalfüllstoffs, bestehend aus Einheiten der Formel

$$n\text{-}C_3H_7SiO_{3/2}\cdot(CH_3)_2SiO_{2/2}\cdot 40\ SiO_2$$

erhalten.

Spezifische Oberfläche: 56 $m^2/g$

| Analysen: | % C | % H | % Si |
|---|---|---|---|
| Theorie: | 2,3 | 0,51 | 45,9 |
| Gefunden: | 2,1 | 0,40 | 44,8 |

Beispiel 5

727,8 g (3,49 Mol) $Si(OC_2H_5)_4$ und 22,2 g (0.1165 Mol) $CH_2$=CH-$Si(OC_2H_5)_3$ wurden miteinander vereinigt. Die Mischung wurde mit 3 ml 0,1 n Essigsäurelösung versetzt und 3 Stunden bei 80° C gerührt.

Nach dieser Vorkondensation wurde die Mischung mit 400 ml Ethanol und mit 200 ml Wasser versetzt und weiter unter Rückfluß gerührt. Der nach 2 Stunden Rückfluß gebildete Feststoff wurde abfiltriert und nach dem Auswaschen mit insgesamt 1 l Wasser analog zu Beispiel 4 weiterbehandelt. Es wurden 216,0 g (98.5 % der Theorie) eines Dentalfüllstoffs, bestehend aus Einheiten der Formel

$$CH_2 = CH\text{-}SiO_{3/2} \cdot 30SiO_2$$

erhalten.

Spezifische Oberfläche: 22 $m^2/g$

| Analysen: | % C | % H | % Si |
|---|---|---|---|
| Theorie: | 1,28 | 0,16 | 46,3 |
| Gefunden: | 1,17 | 0,20 | 45,8 |

Beispiel 6

727,8 g (3.49 Mol) $Si(OC_2H_5)_4$ wurden mit 2 ml 1 %iger wäßriger Ammoniaklösung versetzt und 2 Stunden bei 100° C gerührt. Gleichzeitig wurden 19,0 g (0.07 Mol) $(C_6H_6)_2Si(OC_2H_6)_2$ mit 5 ml Ethanol und 0,5 ml 2 %iger wäßriger Ammoniaklösung versetzt und 10 Stunden bei 100° C gerührt. Anschließend wurden die beiden Vorkondensate vereinigt und mit 500 ml Ethanol sowie 150 ml 2 %iger wäßriger $NH_3$-Lösung versetzt und 3 Stunden weiter unter Rückfluß gerührt. Der gebildete Feststoff wurde abfiltriert und nach dem Auswaschen mit 500 ml Ethanol analog zu Beispiel 4 weiterbehandelt.

Nach einer abschließenden 24-stündigen Temperung bei 150° C unter $N_2$-Atmosphäre wurden 220,0 g (98,1 % der Theorie) eines Dentalfüllstoffs, bestehend aus Einheiten der Formel

$$(C_6H_5)_2SiO_{2/2} \cdot 50SiO_2$$

erhalten.

Spezifische Oberfläche: 31 $m^2/g$

| Analysen: | % C | % H | % Si |
|---|---|---|---|
| Theorie: | 4,50 | 0,31 | 44,7 |
| Gefunden: | 4,40 | 0,26 | 43,9 |

Beispiel 7

17,3 g (0,087 Mol) $(C_6H_5)Si(OCH_3)_3$ wurden mit 0,2 ml 2 %iger methanolischer $NH_3$-Lösung versetzt und zunächst 5 Stunden bei 60° C gerührt. Anschließend wurde das Vorkondensat mit 727,8 g (3.49 Mol) $Si(OC_2H_5)_4$ vereinigt. Nach einer weiteren Verfahrensweise analog zu Beispiel 7 wurden 216, 3 g (98,2 % der Theorie) eines Dentalfüllstoffs, bestehend aus Einheiten der Formel

$$(C_6H_5)SiO_{3/2} \cdot 40SiO_2$$

erhalten.

| | | | |
|---|---|---|---|
| <u>Spezifische Oberfläche:</u> | $48\ m^2/g$ | | |
| <u>Analysen:</u> | % C | % H | % Si |
| Theorie: | 2,85 | 0,20 | 45,5 |
| Gefunden: | 2,95 | 0,31 | 45,8 |

**II. Herstellung der erfindungsgemäßen Dentalwerkstoffe**

Zur Herstellung der erfindungsgemäßen Dentalmassen wurden die Füllstoffe aus den Beispielen Nr. 1 bis Nr. 5 mit einer Kugelmühle auf mittlere Korngrößen zwischen ca. 3 bis 7 µm heruntergemahlen. Dann wurden die Füllstoffe mit 3-Methacryloyloxypropyltrimethoxysilan nach üblichen Verfahren silanisiert.

Die Füllstoffe wurden in Mengen von ca. 70 bis 73 % (m/m) in eine Monomermatrix eingebracht, wie sie für Dentalkunststoffe üblicherweise verwendet wird. Es wurden noch Initiatoren zugesetzt. und die Massen wurden zu homogenen Pasten geknetet.

Es wurde eine Reihe von physikalischen Eigenschaften an aus den verschiedenen Pasten hergestellten ausgehärteten Prüfkörpern bestimmt und mit denen von Handelsprodukten und Laborvergleichsprodukten verglichen (Tabelle I).

**Prüfung und Beurteilung der Polierbarkeit**

Von allen Materialien wurden Prüfkörper eines Durchmessers von 15 mm und einer Dicke von 3 mm hergestellt. Die Oberflächen aller Prüfkörper wurden zunächst mit feinem Schleifpapier (600 grit) gleichmäßig beschliffen. Dann wurden sie unter Wasser mit feinstteiligem Aluminiumoxid (mittlere Teilchengröße 0,04 µm) auf einem Baumwolltuch poliert.

Die Polierbarkeit wurde visuell beurteilt und mittels einer Punkteskala zwischen 1 bis 5 benotet, wobei 1 = matt und 5 = hochglänzend ist.

Beispiele für erfindungsgemäße Dentalmassen

1. Heißhärtende erfindungsgemäße Dentalmassen

Die Herstellung der Prüfkörper aus den heißhärtenden erfindungsgemäßen Dentalmassen erfolgte in der Weise, daß die Massen in die entsprechenden Prüfkörperformen gepreßt und dann in einem Wasserbad unter 6 bar Druck bei 90° C 30 Minuten lang ausgehärtet wurden.

In den folgenden Beispielen werden als Abkürzungen verwendet:

Bis-GMA: 2,2-Bis-[p-(-methacryloyloxy-β-hydroxypropoxy)-phenyl]-propan
TEDMA: Triethylenglykoldimethacrylat

Beispiel 8 (Angaben in Gewichtsteilen):

73,0 Füllstoff gemaß Beispiel 1
18,2 Bis-GMA
8,5 TEDMA
0,3 Dibenzoylperoxid

Beispiel 9 (Angaben in Gewichtsteilen):

70,0 Füllstoff gemäß Beispiel 2
20,3 Bis-GMA
9,4 TEDMA
0,3 Dibenzoylperoxid.

Beispiel 10 (Angaben in Gewichtsteilen):

70,0 Füllstoff gemäß Beispiel 3
20,3 Bis-GMA
9,4 TEDMA
0,3 Dibenzoylperoxid

Beispiel 11 (Angaben in Gewichtsteilen) :

71,0 Füllstoff gemäß Beispiel 4
19,6 Bis-GMA

| | |
|---|---|
| 9,1 | TEDMA |
| 0,3 | Dibenzoylperoxid |

Beispiel 12 (Angaben in Gewichtsteilen):

| | |
|---|---|
| 73,0 | Füllstoff gemäß Beispiel 5 |
| 18,2 | Bis-GMA |
| 8,5 | TEDMA |
| 0,3 | Dibenzoylperoxid |

2. Lichthärtende erfindungsgemäße Dentalmasse

Die lichthärtende erfindungsgemäße Dentalmasse besteht aus einer weißen Paste, die durch Bestrahlung mit einer zahnärztlichen Halogenlichtlampe (Translux, Fa. Kulzer) ausgehärtet wird. Bestrahlungszeit 100 Sekunden

Beispiel 13 (Angaben in Gewichtsteilen):

| | |
|---|---|
| 70,0 | Füllstoff gemäß Beispiel 2 |
| 19,0 | Bis-GMA |
| 8,7 | TEDMA |
| 0,2 | Campherchinon |
| 0,1 | N,N-Dimethyl-p-toluidin |

Handelsprodukte, mit denen die erfindungsgemäßen Dentalmassen in Tabelle I verglichen werden:

Konventionelle Composite (Estilux®, Fa. Kulzer):

Als Füllstoff dient ein silanisiertes Lithiumaluminiumglas einer mittleren Korngröße von ca. 4 µm. Der Füllstoffgehalt liegt bei ca. 75 % (m/m).

Hybrid-Composite (Degufill® H, Degussa):

Als Füllstoff dienen silanisiertes Bariumaluminiumsilikatglas einer mittleren Korngröße von ca. 2 µm, welches aber zu 100 % feiner als 5 µm ist, sowie silanisiertes, hochdisperses $SiO_2$. Der Füllgrad an Glas beträgt ca. 70 % (m/m), der an hochdispersem $SiO_2$ ca. 11 % (m/m). Daraus ergibt sich ein Gesamtgehalt an anorganischen Füllstoffen von ca. 81 % (m/m).

Microfüller Composite (Durafill® , Fa. Kulzer u. Co. GmbH):

Als Füllstoff dient silanisiertes, hochdisperses $SiO_2$ einer mittleren Korngröße zwischen 0.01 bis 0.04 µm. Der Füllgrad beträgt ca. 50 % (m/m).

Die Aushärtung aller Massen erfolgte mit dem Lichtgerät Translux (Fa. Kulzer) und einer Bestrahlungszeit von 40 Sekunden.

Heißhärtende Laborversuchsprodukte = VP

(Angaben in Gewichtsteilen):

| | |
|---|---|
| VP1: | 17 Bis-GMA |
| | 7,7 TEDMA |
| | 75 Bariumaluminiumsilikatglas, silanisiert (mittlere Korngröße ca. 4 µm) |
| | 0,3 Dibenzoylperoxid |
| VP2: | 35 Bis-GMA |
| | 14,7 TEDMA |
| | 50 hochdisperses $SiO_2$, silanisiert (mittlere Korngröße 0,01 - 0,04 µm) |
| | 0,3 Dibenzoylperoxid |

Die Aushärtung dieser Pasten erfolgt analog zur Aushärtung der heißhärtenden erfindungsgemäßen Massen.

Tabelle I: Eigenschaften der erfindungsgemäßen Dentalmassen im Vergleich zu Handelsprodukten sowie zu konventionellen und microgefüllten heißhärtenden Dentalmassen.

| Beispiel | 8 | 9 | 10 | 11 | 12 | 13 | Estilux | Durafill | Degufill H | VP1 | VP2 |
|---|---|---|---|---|---|---|---|---|---|---|---|
| Biegefestigkeit [N/mm²] DIN 13 | 125 | 130 | 80 | 118 | 120 | 90 | 100 | 62 | 110 | 140 | 65 |
| Biegemodul [N/mm²] DIN | 8500 | 9000 | 10700 | 7500 | 8000 | 7600 | 9700 | 322 | 8000 | 11000 | 3900 |
| Druckfestigkeit [N/mm²] | 435 | 400 | 450 | 460 | 470 | 280 | 300 | 350 | 320 | 350 | 410 |
| Vickers Härte HV 5 | 1100 | 860 | 750 | 780 | 820 | 830 | 660 | 290 | 680 | 660 | 330 |
| Polierbarkeit | 5 | 5 | 5 | 5 | 5 | 5 | 1 | 4 | 3 | 1 | 4 |

## Patentansprüche

1. Pastöses, in Gegenwart eines Initiators zu einer auf Hochglanz pollerbaren Masse aushärtbares Dentalmaterial aus einem polymerisierbaren organischen Bindemittel enthaltend mindestens ein polymerisierbares Methacrylat und 20 bis 90 Gew.%, bezogen auf das Gesamtgewicht eines feinteiligen Füllstoffs aus einem Organosiloxan-Copolykondensat, welches als statistisches Copolykondensat, Block-Copolykondensat oder als Gemisch aus diesen Formen vorliegt und eine spezifische Oberfläche von bis zu 200 $m^2/g$ und eine Partilkelgröße von 0,01 μm bis 100 μm aufweist und aus Einheiten der Formeln

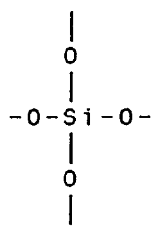

(I)

und

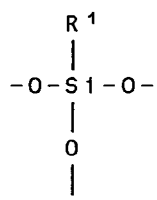

(II)

wobei $R^1$ für eine mit einem Acrylat- oder Methacrylatrest verbundene lineare oder verzweigte Alkylgruppe mit 1 bis 6 C-Atomen oder für einen einfach olefinisch ungesättigten, vorzugsweise endständig ungesättigten linearen, gegebenenfalls verzweigten Kohlenwasserstoffrest mit 2 bis 8 C-Atomen oder für einen zyklischen, einfach olefinisch ungesättigten Kohlenwasserstoffrest mit 5 - 8 C-Atomen oder für eine lineare, gegebenenfalls verzweigte Alkylgruppe mit 1 bis 8 C-Atomen, eine Cycloalkylengruppe mit 5 bis 8 C-Atomen, eine Phenylgruppe oder eine Alkylarylgruppe steht und/oder Einheiten der Formel

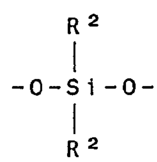

(III)

in denen $R^2$ eine Methyl-, Ethyl- oder Phenylgruppe repräsentiert, aufgebaut ist und die freien Valenzen der an die Siliciumatome gebundenen Sauerstoffatome bei den Einheiten (I), (II) und (III) wie bei den Kieselsäuregerüsten durch ein Siliciumatom einer gleichen oder einer unterschiedlichen Einheit abgesättigt sind, wobei das Verhältnis der Siliciumatome aus den Einheiten der Formel (I) zu der Summe der Siliciumatome der Einheiten (II) und (III) 3 : 1 bis 100 : 1 beträgt und das erhältlich ist

A) in Gestalt eines statistischen Copolykondensats, indem man

a) ein Alkoxysilan der allgemeinen Formel

$$Si\,(OR^3)_4 \qquad (IV)$$

wobei $R^3$ für eine lineare oder verzweigte Alkylgruppe mit 1 bis 5 C-Atomen steht und ein Alkoxysilan der allgemeinen Formel

$$R^1 - Si(OR^3)_3 \qquad (V)$$

in der $R^1$ wie unter Formel (II) und $R^3$ wie unter Formel (IV) definiert sind und/oder ein Alkoxysilan der allgemeinen Formel

$$R^2_2Si(OR^3)_2 \qquad (VI)$$

in der $R^2$ wie unter Formel (III) und $R^3$ wie unter Formel (IV) definiert sind, in einem weitgehend wassermischbaren, aber die Silane nach Formeln (IV) bis (VI) lösenden Lösungsmittel auflöst und der Lösung unter Rühren eine zumindest für die vollständige Hydrolyse und Kondensation ausreichende Menge Wasser zufügt und das Reaktionsgemisch unter Weiterrühren bei einer bestimmten Temperatur im Bereich von Raumtemperatur bis 200° C vorkondensiert,
b) den sich bildenden Feststoff gegebenenfalls nach Zusatz weiteren Lösungsmittels oder Wassers noch 1 Stunde bis zu 6 Stunden bei 60° C bis 200° C, bei Normaldruck oder einem Druck rührt, welcher der Summe der Partialdrucke bei der jeweiligen Temperatur entspricht,
c) das als Feststoff gebildete Organopolysiloxan nach gängigen Techniken von der flüssigen Phase abtrennt, gegebenenfalls wäscht,
d) dann in Wasser, einem Wasser/Alkohol-Gemisch oder in reinem Alkohol, in Gegenwart eines sauren oder eines basischen Katalysators nachkondensiert und
e) gegebenenfalls bei Raumtemperatur bis 200° C, gegebenenfalls unter Schutzgasatmosphäre oder im Vakuum trocknet, gegebenenfalls 1 bis 100 Stunden bei Temperaturen von 150° C bis 250° C unter Schutzgasatmosphäre oder im Vakuum tempert und gegebenenfalls mahlt und/oder klassifiziert.

B) in Gestalt eines Block-Copolykondensats, indem man
a) die monomeren Komponenten nach Formel (IV), (V), und/oder (VI) jeweils unabhängig voneinander, ohne oder unter Einsatz eines die Ausgangsstoffe lösenden Lösungsmittels in Gegenwart einer nicht zur vollständigen Hydrolyse ausreichenden Menge Wasser über einen Zeitraum von 5 Minuten bis zu 5 Tagen bei Raumtemperatur bis 200° C vorkondensiert, die erhaltenen Vorkondensate vereinigt und gegebenenfalls nach Zusatz weiteren Wassers und/oder weiteren Lösungsmittels die unter A) genannten Schritte b) bis e) anschließt oder

C) in Gestalt eines gemischten Copolykondensats, indem man
a) von den monomeren Komponenten nach Formel (IV) (V) und/oder (VI) mindestens ein Monomer aber höchstens 2 Monomere, voneinander unabhängig wie bei B) vorkondensiert, das erhaltene Vorkondensat oder die erhaltenen Vorkondensate und mindestens eine nicht vorkondensierte Komponente miteinander vereinigt und die unter A) genannten Schritte b) bis e) anschließt,

**dadurch gekennzeichnet,**
daß man bei der Herstellung der Füllstoffe, die Nachkondensation im Autoklaven über einen Zeitraum von 1 Stunde bis zu 5 Tagen bei Temperaturen von 60 bis 250° unter einem Druck, welcher der Summe der Partialdrucke bei der jeweiligen Temperatur entspricht, durchführt.

**2.** Dentalmaterial nach Anspruch 1,
**dadurch gekennzeichnet,**
daß der feinteilige füllstoff aus dem Organosiloxan-Copolykondensat in einer Menge von 50 bis 85 Gew.-%, bezogen auf das Gesamtgewicht, vorliegt.

**3.** Dentalmaterial gemäß Anspruch 1,
**dadurch gekennzeichnet,**
daß $R^1$ in Formel (II) für die Gruppe

$$-(CH_2)_3-O-\overset{\overset{\displaystyle O}{\|}}{C}-\overset{\overset{\displaystyle CH_3}{|}}{C}=CH_2$$

steht.

**4.** Dentalmaterial gemäß Anspruch 1,
**dadurch gekennzeichnet,**
daß es als Füllstoff ein Organopolysiloxan enthält, das aus Einheiten der Formel (I) und Einheiten der Formel (II) mit der Zusammensetzung

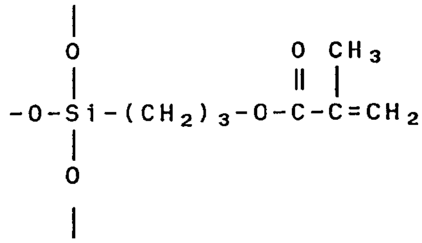

besteht, wobei das molare Verhältnis der Einheiten nach Formel (I) zu den Einheiten der Formel (II) 3 : 1 bis 100 : 1 beträgt.

5. Dentalmaterial gemäß Anspruch 1,
**dadurch gekennzeichnet,**
daß es als Füllstoff ein Organopolysiloxan enthält, das aus Einheiten der Formel (I) und Einheiten der Formel (III) mit der Zusammensetzung

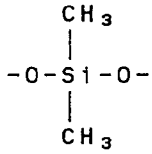

besteht, wobei das molare Verhältnis der Einheiten nach Formel (I) zu den Einheiten der Formel (III) 3 : 1 bis 100 : 1 beträgt.

6. Dentalmaterial gemäß den Ansprüchen 1 bis 4,
**dadurch gekennzeichnet,**
daß sein Füllstoff in Gestalt eines statistischen Copolykondensats erhältlich ist, indem Hydrolyse und Kondensation in Methanol, Ethanol, n- und i-Propanol, n- und i-Butanol und/oder n-Pentanol durchgeführt werden.

7. Dentalmaterial nach Anspruch 1,
**dadurch gekennzeichnet,**
daß man zur Herstellung eines Block-Copolykondensats oder eines gemischten Copolykondensats die Ausgangsstoffe in einem zu deren Alkoxygruppen korrespondierenden, linearen oder verzweigten Alkohol mit 1 bis 5 C-Atomen auflöst.

8. Dentalmaterial nach Anspruch 1,
**dadurch gekennzeichnet,**
daß man bei der Herstellung eines Block-Copolykondensats oder eines gemischten Copolykondensats im Schritt a) Wasser in einer Menge von 1 bis 100 Mol% der zur vollständigen Hydrolyse benötigten Menge einsetzt.

9. Dentalmaterial gemäß den Anprüchen 1 bis 5,
**dadurch gekennzeichnet,**
daß sein Füllstoff in Gestalt eines statistischen Copolykondensats erhältlich ist, indem man die monomeren Komponenten nach Formel (IV), (V) und/oder (VI) ohne oder unter Einsatz eines die Ausgangsstoffe lösenden Lösungsmittels, bevorzugt eines zu den Alkoxygruppen korrespondierenden linearen oder verzweigten Alkohols mit 1 bis 5 C-Atomen, in Gegenwart einer nicht zur vollständigen Hydrolyse ausreichenden Menge Wasser, vorzugsweise 1 bis 100 Mol% der hierzu benötigten Menge, über einen Zeitraum von 5 Minuten bis zu 5 Tagen bei Raumtemperatur bis 200° C vorkondensiert.

10. Dentalmaterial gemäß Ansprüchen 1 bis 6,
**dadurch gekennzeichnet,**
daß sein Füllstoff erhältlich ist, Indem man zur Vorkondensation einen sauren, basischen und/oder metallhaltigen Kondensationskatalysator anwendet.

**11.** Dentalmaterial nach Anspruch 1,
**dadurch gekennzeichnet,**
daß man bei der Herstellung des Copolykondensats als basischen Katalysator für die Nachkondensation im Schritt d) Ammoniak einsetzt.

**12.** Verwendung des Dentalmaterials nach vorstehenden Ansprüchen zur Fertigung von Zahnfüllungen, Inlays, Verblendungen, Zahnversiegelungen, Überzügen zum Schutz von Zahnoberflächen, Kronen, Brücken, Zahnprothesen, künstlichen Zähnen, Klebern zur Befestigung von Inlays, Kronen und Brücken sowie zum Aufbau von Zahnstümpfen.

## Claims

**1.** Paste-like dental material which, in the presence of an initiator, may be cured into a composition polishable to a high gloss, and which is prepared from a polymerisable organic binder containing at least one polymerisable methacrylate and 20 to 90 wt.%, related to the total weight, of a finely divided filler prepared from an organosiloxane copolycondensate which is present as a random copolycondensate, block copolycondensate or as a mixture of these forms and has a specific surface area of up to 200 $m^2/g$ and a particle size from 0.01 $\mu m$ to 100 $\mu m$, and is composed of units of the formulae

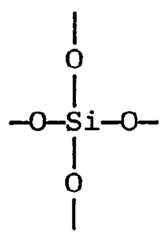

(I)

and

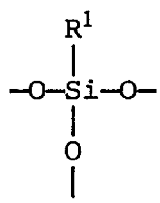

(II)

where $R^1$ stands for a linear or branched alkyl group bonded to an acrylate or methacrylate radical and having 1 to 6 C atoms, or for a singly olefinically unsaturated, preferably terminally unsaturated, linear, optionally branched, hydrocarbon radical with 2 to 8 C atoms or for a cyclic, singly olefinically unsaturated hydrocarbon radical with 5 to 8 C atoms or for a linear, optionally branched, alkyl group with 1 to 8 C atoms, a cycloalkene group with 5 to 8 C atoms, a phenyl group or an alkylaryl group, and/or units of the formula

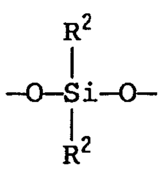

(III)

in which $R^2$ represents a methyl, ethyl or phenyl group and the free valencies of the oxygen atoms bonded to the silicon atoms in units (I), (II) and (III) are saturated, as in silica skeletons, by a silicon atom of an identical or different unit, the ratio of the silicon atoms from the units of formula (I) to the sum of the silicon atoms from units (II) and (III) being 3:1 to 100:1, and is obtainable

A) in the form of a random copolycondensate by

a) dissolving an alkoxysilane of the general formula

$$Si(OR^3)_4 \qquad (IV)$$

where $R^3$ stands for a linear or branched alkyl group with 1 to 5 C atoms, and an alkoxysilane of the general formula

$$R^1\text{-}Si(OR^3)_3 \qquad (V)$$

in which $R^1$ is defined as under formula (II) and $R^3$ as under formula (IV), and/or an alkoxysilane of the general formula

$$R^2{}_2Si(OR^3)_2 \qquad (VI)$$

in which $R^2$ is defined as under formula (III) and $R^3$ as under formula (IV), in a largely water-miscible solvent, which does however dissolve the silanes according to formulae (IV) to (VI), and adding to the solution while stirring a quantity of water at least sufficient for complete hydrolysis and condensation, and precondensing the reaction mixture with continued stirring at a specific temperature in the range from room temperature to 200°C,

b) stirring the solid which is forming, optionally after the addition of further solvent or water, for a further 1 hour to 6 hours at 60° to 200°C at standard pressure or a pressure corresponding to the sum of partial pressures at the temperature concerned,

c) separating the organopolysiloxane formed as a solid from the liquid phase using conventional techniques, optionally washing,

d) then post-condensing in water, a water/alcohol mixture or in pure alcohol in the presence of an acid or basic catalyst and

e) optionally drying at room temperature to 200°C, optionally under a protective gas atmosphere or a vacuum, optionally conditioning for 1 to 100 hours at temperatures of 150°C to 250°C under a protective gas atmosphere or a vacuum and optionally grinding and/or classifying.

B) in the form of a block copolycondensate by

a) precondensing the monomeric components according to formulae (IV), (V) and/or (VI) each mutually independently with or without the use of a solvent dissolving the starting materials, in the presence of an amount of water insufficient for complete hydrolysis, over a period from 5 minutes to 5 days at room temperature to 200°C, combining the precondensates obtained and then, optionally after the addition of further water and/or further solvent, performing stages b) to e) stated under A) or

C) in the form of a mixed copolycondensate by

a) precondensing at least one monomer, but at most two monomers, from the monomeric components according to formulae (IV), (V) and/or (VI) mutually independently as in B), combining the precondensate(s) obtained with at least one non-precondensed component and then performing stages b) to e) stated under A),

characterised in that,
during production of the fillers, post-condensation is performed in an autoclave over a period from 1 hour to 5 days at temperatures from 60 to 250°C at a pressure corresponding to the sum of partial pressures at the temperature concerned.

2. Dental material according to claim 1,
characterised in that
the finely divided filler prepared from the organosiloxane copolycondensate is present in a quantity of 50 to 85 wt.%, related to the total weight.

3. Dental material according to claim 1,
characterised in that
$R^1$ in formula (II) stands for the group

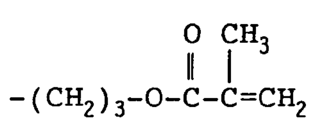

4. Dental material according to claim 1,
characterised in that
it contains as filler an organopolysiloxane which comprises units of the formula (I) and units of the formula (II) with the composition

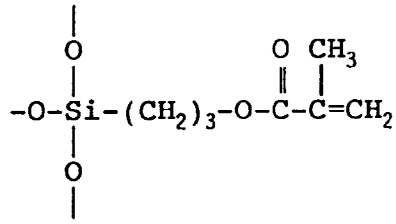

where the molar ratio of the units according to formula (I) to the units of formula (II) is 3:1 to 100:1.

**5.** Dental material according to claim 1,
characterised in that
it contains as filler an organopolysiloxane which comprises units of the formula (I) and units of the formula (III) with the composition

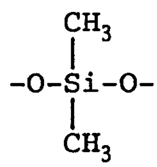

where the molar ratio of the units according to formula (I) to the units of formula (II) is 3:1 to 100:1.

**6.** Dental material according to claims 1 to 4,
characterised in that
its filler is obtainable in the form of a random copolycondensate by performing hydrolysis and condensation in methanol, ethanol, n- and i- propanol, n- and i- butanol and/or n-pentanol.

**7.** Dental material according to claim 1,
characterised in that,
in order to produce a block copolycondensate or a mixed copolycondensate, the starting materials are dissolved in a linear or branched alcohol with 1 to 5 C atoms corresponding to their alkoxy groups.

**8.** Dental material according to claim 1,
characterised in that,
during the production of a block copolycondensate or a mixed copolycondensate, water is added in stage a) in a quantity of 1 to 100 mol% of the quantity required for complete hydrolysis.

**9.** Dental material according to claims 1 to 5,
characterised in that
its filler may be obtained in the form of a random copolycondensate by precondensing the monomeric components according to formulae (IV), (V) and/or (VI), with or without the use of a solvent which dissolves the starting materials, preferably a linear or branched alcohol with 1 to 5 C atoms corresponding to the alkoxy groups, in the presence of a quantity of water which is insufficient for complete hydrolysis, preferably from 1 to 100 mol% of the quantity required therefor, over a period of 5 minutes to 5 days at room temperature to 200°C.

**10.** Dental material according to claims 1 to 6,
characterised in that
its filler may be obtained by using for precondensation a condensation catalyst which is acid, basic and/or contains metal.

**11.** Dental material according to claim 1,
characterised in that,
during production of the copolycondensate, ammonia is used as the basic catalyst for post-condensation in stage d).

12. Use of the dental material according to the preceding claims for the preparation of dental fillings, inlays, overlays, dental sealants, coatings to protect tooth surfaces, crowns, bridges, dental prostheses, artificial teeth, adhesives for the attachment of inlays, crowns and bridges and for building up tooth stumps.

## Revendications

1. Matériau dentaire pâteux, durcissable en présence d'un initiateur en une masse qui peut être polie, à brillance élevée, à partir d'un agent de liaison organique polymérisable, qui renferme au moins un méthacrylate polymérisable et de 20 à 90 % en poids rapporté au poids total, d'une substance de charge finement divisée, à base d'un copolycondensat d'organosiloxane, qui se présente sous forme de copolycondensat statistique, de copolycondensat en blocs, ou sous forme de mélange de ces formes et qui possède une surface spécifique allant jusqu'à 200 $m^2/g$ et une taille de particules allant de 0,01 μm à 100 μm, et qui est construite de motifs de formules

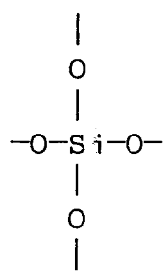

(I)

et

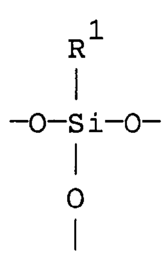

(II)

dans lesquelles $R^1$ représente un radical alcoyle ayant de 1 à 6 atomes de carbone linéaire ou ramifié lié à un reste acrylate ou méthacrylate, ou un radical hydrocarboné non saturé une fois oléfinique, de préférence linéaire avec une insaturation terminale, le cas échéant ramifié ayant de 2 à 8 atomes de carbone, ou un radical hydrocarboné non saturé cyclique une fois oléfinique ayant de 5 à 8 atomes de carbone, ou un radical alcoyle linéaire ou éventuellement ramifié ayant de 1 à 8 atomes de carbone, un radical cycloalcoylène ayant de 5 à 8 atomes de carbone, un radical phényle, ou un radical alcoylaryle et/ou de motifs de formule

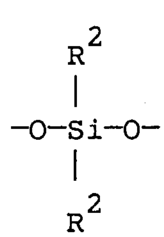

(III)

dans laquelle $R^2$ représente un radical méthyle, éthyle ou phényle et
les valences libres des atomes d'oxygène liés aux atomes de silicium dans les motifs (I) (II) et (III) comme dans les squelettes d'acide silicique, sont saturées par un atome de silicium d'un motif identique ou différent, le rapport des atomes de silicium dans les motifs de formule (I) à la somme des atomes de silicium

des motifs (II) et (III) s'élèvant de 3 : 1 à 100 : 1,
et celui-ci est disponible

A) Sous forme d'un copolycondensat statistique dans lequel

a) on dissout un alcoxysilane de formule générale

$$Si\,(OR^3)_4 \qquad (IV)$$

dans laquelle $R^3$ représente un radical alcoyle ayant de 1 à 5 atomes de carbone, linéaire ou ramifié et un alcoxysilane de formule générale

$$R^1 - Si(OR^3)_3 \qquad (V)$$

dans laquelle $R^1$ est défini comme à la formule (II) et $R^3$ est défini comme dans la formule (IV) et/ou un alcoxysilane de formule générale

$$R^2_2Si(OR^3)_2 \qquad (VI)$$

dans laquelle $R^2$ est défini comme dans la formule (III) et $R^3$ est défini comme dans la formule (IV) dans un solvant largement miscible à l'eau, mais qui dissout les silanes selon les formules (IV) à (VI)

et on ajoute à la solution sous agitation une quantité d'eau suffisante au moins pour l'hydrolyse complète et pour la condensation, et on pré-condense le mélange réactionnel tout en poursuivant l'agitation, à une température déterminée dans la plage allant de la température ambiante jusqu'à 200°C,

b) on agite le solide qui s'est formé, le cas échéant, après ajout de davantage de solvant ou d'eau, encore pendant une heure jusqu'à six heures à une température qui va de 60 à 200°C, à pression ordinaire ou sous pression qui correspond à la somme des pressions partielles à la température correspondante,

c) on sépare l'organopolysiloxane formé sous forme de solide, selon les techniques courantes de la phase liquide et le cas échéant on lave,

d) ensuite on condense finalement dans l'eau, dans un mélange eau/alcool ou dans l'alcool pur en présence d'un catalyseur acide ou basique et,

e) le cas échéant, on sèche le cas échéant à une température allant de la température ambiante à 200°C, éventuellement sous atmosphère de gaz protecteur ou sous vide, on recuit le cas échéant pendant 1 à 100 heures à des températures comprises entre 150 et 250°C sous atmosphère de gaz protecteur ou sous le vide et éventuellement on broye et/ou on classifie.

B) Sous forme d'un copolycondensat en blocs dans lequel

a) on pré-condense les composants monomériques selon les formules (IV), (V), et/ou (VI) respectivement indépendamment l'un de l'autre, sans ou avec utilisation d'un solvant qui dissout les substances de départ en présence d'une quantité d'eau qui n'est pas suffisante pour l'hydrolyse complète pendant un espace de temps qui va de 5 minutes à 5 jours, à des températures allant de la température ambiante à 200°C, on réunit les pré-condensats obtenus et on enchaîne, le cas échéant, après addition de davantage d'eau et/ou de davantage de solvant, les étapes b) à e) mentionnées sous A) ou

C) Sous la forme d'un copolycondensat mixte dans lequel

a) on pré-condense parmi les composants monomériques selon les formules (IV), (V) et/ou (VI) au moins un monomère mais au plus deux monomères, indépendamment l'un de l'autre, comme pour (B), on réunit le pré-condensat obtenu ou les pré-condensats obtenus et au moins un composant non pré-condensé, les uns avec les autres et on enchaîne les étapes b) à e) mentionnées sous A), caractérisé en ce que l'on

effectue lors de la production des substances de charge, la condensation finale dans un autoclave pendant un espace de temps allant de une heure à 5 jours, à des températures allant de 60 à 250°C sous une pression qui correspond à la somme des pressions partielles à la température correspondante.

**2.** Matériau dentaire selon la revendication 1, caractérisé en ce que la substance de charge finement divisée à base du copolycondensat d'organosiloxane, est présente en quantité allant de 50 à 85 % en poids, rapporté au poids total.

**3.** Matériau dentaire selon la revendication 1, caractérisé en ce que $R^1$ représente dans la formule (II) le groupe

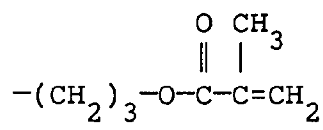

4. Matériau dentaire selon la revendication 1, caractérisé en ce qu'il contient comme substance de charge un organopolysiloxane, qui consiste en des motifs de formule (I) et en des motifs de formule (II), ayant la composition

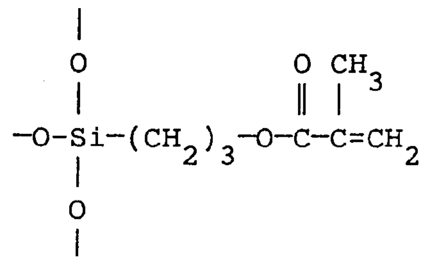

dans lequel le rapport molaire des motifs selon la formule (I) aux motifs de formule (II) s'élève de 3 : 1 à 100 : 1.

5. Matériau dentaire selon la revendication 1, caractérisé en ce qu'il contient comme substance de charge, un organopolysiloxane qui consiste en des motifs de formule (I) et en des motifs de formule (III) ayant la composition

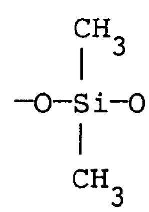

dans lequel le rapport molaire des motifs selon la formule (I) aux motifs de formule (III) s'élève de 3 : 1 à 100 : 1.

6. Matériau dentaire selon les revendications 1 à 4, caractérisé en ce que la substance de charge est disponible sous forme d'un copolycondensat statistique dans lequel l'hydrolyse et la condensation sont effectuées dans le méthanol, l'éthanol, le n- et le i-propanol, le n- et le i-butanol, et/ou le n-pentanol.

7. Matériau dentaire selon la revendication 1, caractérisé en ce que l'on dissout pour l'obtention d'un copolycondensat en blocs ou d'un copolycondensat mixte, les produits de départ dans un alcool ayant de 1 à 5 atomes de carbone, linéaire ou ramifié, qui correspond à ses groupes alcoxy.

8. Matériau dentaire selon la revendication 1, caractérisé en ce que l'on met en oeuvre pour l'obtention d'un copolycondensat en blocs ou d'un copolycondensat mixte, à l'étape a) de l'eau en une quantité de 1 à 100 % molaire de la quantité nécessaire pour une hydrolyse complète.

9. Matériau dentaire selon les revendications 1 à 5, caractérisé en ce que sa substance de charge est disponible sous forme d'un copolycondensat statistique, dans laquelle on pré-condense les composants monomériques selon les formules (IV), (V) et/ou (VI) sans ou avec ajout d'un solvant qui dissout les produits de départ, de préférence d'un alcool linéaire ou ramifié ayant de 1 à 5 atomes de carbone, qui correspond aux groupes alcoxy, en présence d'une quantité d'eau qui n'est pas suffisante pour l'hydrolyse complète, de préférence de 1 à 100 % molaire de la quantité nécessaire pour cela, pendant un espace de temps allant de 5 minutes à 5 jours à une température allant de la température ambiante à 200°C.

10. Matériau dentaire selon les revendications 1 à 6, caractérisé en ce qua sa substance de charge est disponible, en utilisant pour la pré-condensation un catalyseur de condensation acide, basique et/ou renfermant un métal.

11. Matériau dentaire selon la revendication 1, caractérisé en ce que l'on met en oeuvre pour la production du copolycondensat à l'étape d) de l'ammoniac comme catalyseur basique pour la condensation finale.

12. Utilisation du matériau dentaire selon les revendications précédentes pour la finition d'obturations de dents, de recouvrements (Inlay) de plombages, de scellements de dents, de recouvrements pour la protection de surfaces dentaires, de couronnes, de bridges, de prothèses dentaires, de dents artificielles, de colles pour la fixation de recouvrements (Inlay), de couronnes, et de bridges, ainsi que pour la reconstruction de moignons de dents.